# EUROPEAN PATENT APPLICATION

(11) **EP 1 683 523 A1**
(43) Date of publication of application: **26.07.2006**
(21) Application number: 05001419.0
(22) Date of filing: 25.01.2005
(51) Int. Cl.: A61K 31/498, C07D 241/42, A61P 35/00

(54) **2-Phenylquinoxalines as inhibitors for MPP1**

(71) Applicant: MAX-PLANCK-GESELLSCHAFT ZUR FÖRDERUNG DER WISSENSCHAFTEN E.V., 80539 München (DE)
(72) Inventor: Nigg, Erich A, 80805 Munich (DE); Mayer, Thomas U, 81375 Munich (DE); Hümmer, Stefan, 82152 Planegg (DE); Barr, Francis, 81245 Munich (DE); Bormann, Jenny, 81547 Munich (DE)
(74) Representative: Arth, Hans-Lothar

(57) **Abstract**

The present invention relates to 2-phenylquinoxaline derivatives as well as pharmaceutical compositions containing at least one of these 2-phenylquinoxaline derivatives together with pharmaceutically acceptable carrier, excipient and/or diluents. Said 2-phenylquinoxaline derivatives have been identified as specific inhibitors of the mitotic kinesin MPP1, and are useful for the treatment of cancers and proliferative diseases including tumor growth and metastases.

## Description

The present invention relates to 2-phenylquinoxaline derivatives and stereoisomeric forms, prodrugs, solvates, hydrates and/or pharmaceutically acceptable salts of these compounds as well as pharmaceutical compositions containing at least one of these 2-phenylquinoxaline derivatives together with pharmaceutically acceptable carrier, excipient and/or diluents. Said 2-phenylquinoxaline derivatives have been identified as specific inhibitors of the mitotic kinesin MPP1, and are useful for the treatment of cancers and proliferative diseases including tumor growth and metastases.

### Background of the invention

The mechanism by which the cell coordinates the division of the chromosomes (mitosis) and division of the cytoplasm (cytokinesis) remains among the largest unsolved problems in cell biology. Establishment, maintenance and the concerted contraction of the actomyosin structures of the cleavage furrow are continuously regulated by the microtubules of the anaphase spindle and telophase midbody. Many molecules are known to be involved in cytokinesis, but the spatial as well as the temporal regulation and mechanistic roles of the vast majority of these proteins remain unclear. Previously, it has been shown that the activities of the kinesins Mklp1, Mklp2, and MPP1 are essential for cytokinesis. Mklp1 and Mklp2 have been shown to regulate the localization of RhoGAP and Polo kinase to the spindle midzone, respectively. These data suggest that Mklp1 and Mklp2 have regulatory functions during cytokinesis. While RNA intereference studies revealed that MPP1 is required late in cytokinesis its exact function remains unknown.

It is object of the present invention to provide compounds and/or pharmaceutically acceptable salts thereof which can be used as pharmaceutically active agents, especially for prophylaxis and/or treatment of proliferative diseases such as cancers.

The object of the present invention is solved by the teaching of the independent claims. Further advantageous features, aspects and details of the invention are evident from the dependent claims, the description, and the examples of the present application.

The 2-phenylquinoxaline derivatives according to the present invention are represented by the following general formula (I) 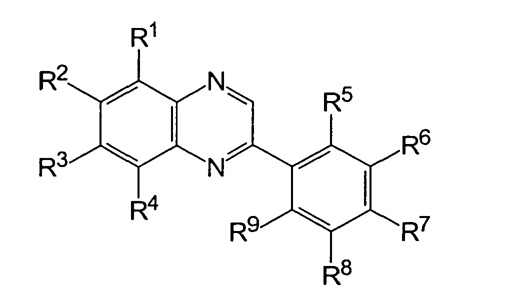 wherein
R¹, R², R³, R⁴, R⁵, R⁸, and R⁹ are independently of each other selected from R¹⁰ - R¹⁸, -CH₂-R¹⁹, -C₂H₄-R²⁰, -C₃H₆-R²¹, -C₄H₈-R²², -C₅H₁₀-R²³, -C₆H₁₂-R²⁴, -C₇H₁₄-R²⁵ -CHR²⁶R²⁷ -CR²⁸R²⁹R³⁰, -CHR³¹-CHR³²R³³, -CR³⁴R³⁵-CR³⁶R³⁷R³⁸ -CHR³⁹-CHR⁴⁰-CHR⁴¹R⁴², -CR⁴³R⁴⁴-CR⁴⁵R⁴⁶-CR⁴⁷R⁴⁸R⁴⁹.
R¹⁰ - R⁴⁹ represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NH-cyclo-C₃H₅, -NH-CS-NHC₃H₇, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-C(=NH)-NH₂, -NH-CS-NHC₂H₅, -NH-CS-NHCH₃, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -0-CO-OC₃H₇, -O-CO-O-cyclo-C₃H₅, -O-CO-OCH(CH₃)₂, -O-CO-OC(CH₃)₃, -CH₂F -CHF₂, -CF₃, -CH₂Cl, -CHCl₂, -CCl₃, -CH₂Br -CHBr₂, -CBr₃, -CPh₃, -CH₂-CH₂F -CH₂-CHF₂, -CH₂-CF₃, -CH₂-CH₂Cl, -CH₂-CHCl₂, -CH₂-CCl₃, -CH₂-CH₂Br -CH₂-CHBr₂, -CH₂-CBr₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH₂-C(CH₃)₂-C₂H₅, -CH(CH₃)-CH(CH₃)-C₂H₅, -CH₂-CH(CH₃)-CH(CH₃)₂, -C(CH₃)₂-C₃H₇, -C(CH₃)₂-CH(CH₃)₂, -C₂H₄-C(CH₃)₃, -CH(CH₃)-C(CH₃)₃, -C₇H₁₅, -C₈H₁₇, -C₉H₁₉, -C₁₀H₂₁, -Ph, -CH₂-Ph, -C₂H₄-Ph, -C₂H₂-Ph, -C≡C-Ph, -CPh₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -C≡CH, -CH=CH-C₃H₇, -C≡C-CH₃, -CH₂-C≡CH, -C₂H₄-C≡CH, -CH₂-C≡C-CH₃, -C≡C-C₂H₅, -C₃H₆-C≡CH, -cyclo-C₃H₅, -cyclo-C₄H₇, -cyclo-C₅H₉, -cyclo-C₆H₁₁;
R⁶ and R⁷ represent -Cl; and
at least one of the substituents R⁵, R⁸, and R⁹ is different from hydrogen; and stereoisomeric forms, prodrugs, solvates, hydrates and/or pharmaceutically acceptable salts thereof.

Preferred are the 2-phenylquinoxaline derivatives wherein R³ represents -CF₃.

Also preferred are the 2-phenylquinoxaline derivatives wherein R¹ represents -Cl.

The above-mentioned 2-phenylquinoxaline derivatives are especially useful as pharmaceutically active agents, most especially for prophylaxis and/or treatment of proliferative diseases and cancers.

Thus, the present invention relates to the use of 2-phenylquinoxaline derivatives of the general formula (I) 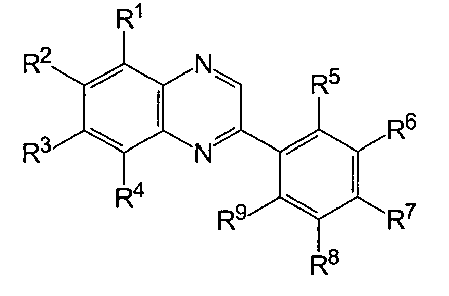 wherein
R¹, R², R³, R⁴, R⁵, R⁶, R⁷, R⁸, and R⁹ are independently of each other selected from R¹⁰ - R¹⁸, -CH₂-R¹⁹, -C₂H₄-R²⁰, -C₃H₆-R²¹, -C₄H₈-R²², -C₅H₁₀-R²³, -C₆H₁₂-R²⁴, -C₇H₁₄-R²⁵, -CHR²⁶R²⁷, -CR²⁸R²⁹R³⁰, -CHR³¹-CHR³²R³³, -CR³⁴R³⁵-CR³⁶R³⁷R³⁸, -CHR³⁹ -CHR⁴⁰ -CHR⁴¹R⁴², -CR⁴³R⁴⁴-CR⁴⁵R⁴⁶-CR⁴⁷R⁴⁸R⁴⁹, R¹⁰ - R⁴⁹ represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NH-cyclo-C₃H₅, -NH-CS-NHC₃H₇, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂. -NH-CS-N(cyclo-C₃H₅)₂, -NH-C(=NH)-NH₂, -NH-CS-NHC₂H₅, -NH-CS-NHCH₃, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F -CHF₂, -CF₃, -CH₂Cl, -CHCl₂, -CCl₃, -CH₂Br -CHBr₂, -CBr₃, -CPh₃, -CH₂-CH₂F -CH₂-CHF₂, -CH₂-CF₃, -CH₂-CH₂Cl, -CH₂-CHCl₂, -CH₂-CCl₃, -CH₂-CH₂Br -CH₂-CHBr₂, -CH₂-CBr₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH₂-C(CH₃)₂-C₂H₅, -CH(CH₃)-CH(CH₃)-C₂H₅, -CH₂-CH(CH₃)-CH(CH₃)₂, -C(CH₃)₂-C₃H₇, -C(CH₃)₂-CH(CH₃)₂, -C₂H₄-C(CH₃)₃, -CH(CH₃)-C(CH₃)₃, -C₇H₁₅, -C₈H₁₇, -C₉H₁₉, -C₁₀H₂₁, -Ph, -CH₂-Ph, -C₂H₄-Ph, -C₂H₂-Ph, -C≡C-Ph, -CPh₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -C≡CH, -CH=CH-C₃H₇, -C≡C-CH₃, -CH₂-C≡CH, -C₂H₄-C≡CH, -CH₂-C≡C-CH₃, -C≡C-C₂H₅, -C₃H₆-C≡CH, -cyclo-C₃H₅, -cyclo-C₄H₇, -cyclo-C₅H₉, -cyclo-C₆H₁₁;
and stereoisomeric forms, prodrugs, solvates, hydrates and/or pharmaceutically acceptable salts thereof for prophylaxis and/or treatment of cancers and proliferative diseases.

The present invention also comprises pharmaceutically acceptable salts of the compounds according to the general formula (I), all stereoisomeric forms of the compounds according to the general formula (I) as well as solvates, especially hydrates or prodrugs thereof. A prodrug is commonly described as an inactive or protected derivative of an active ingredient or a drug, which is converted to the active ingredient or drug in the body.

The 2-phenylquinoxaline derivatives are basic and may form salts with organic or inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner. In case, the compounds bear acidic substituents, the formation of salts with inorganic or organic bases may be possible. Examples for such bases are NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Some compounds of the general formula (I) may exist in the form of optical isomers, e.g. enantiomers, diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1% w/w of the other isomer(s). The afore-mentioned compounds are useful as pharmaceutically active agents, i.d. as drugs or medicine.

The 2-phenylquinoxaline derivatives are specific inhibitors of the mitotic kinesin MPP1. The 2-phenylquinoxaline derivatives were identified as MPP1 inhibitors be means of a malachite-green based in vitro ATPase assay. The human M-Phase Phosphoprotein 1 (MPP1), previously identified through a screening of a subset of proteins specifically phosphorylated at the G2/M transition, is characterized as a plus-end-directed kinesin-related protein. Recombinant MPP1 exhibits in vitro microtubule-binding and microtubule-bundling properties as well as microtubules-stimulated ATPase activity. ln gliding experiments using polarity-marked microtubules, MPP1 is a slow molecular motor that moves towards the microtubule plus-end at a 0.07 µm/s speed. In cycling cells, MPP1 localizes mainly to the nuclei in interphase. During mitosis, MPP1 is diffuse throughout the cytoplasm in metaphase and subsequently localizes to the midzone to further concentrate on the midbody. MPP1 suppression by RNA interference induces failure of cell division late in cytokinesis. It is assumed that MPP1 is a new mitotic molecular motor required for completion of cytokinesis.

Concerning the function of MPP1 it is suggested that inhibitors of MPP1 are useful for the treatment of any kind of cancer since MPP1 seems to be essential for mitosis.

Preferred as MPP1 inhibitors and preferred for prophylaxsis and/or treatment of proliferative diseases are the compounds of general formula (I) wherein R⁶ is a chloro substituent. Also preferred are the 2-phenylquinoxaline derivatives wherein R⁷ is a chloro substituent. Still preferred are the 2-phenylquinoxaline derivatives wherein R¹ is a chloro substituent. Preferred are also the compounds of general formula (I) wherein R³ represents -CF₃ group.

More preferred are the compounds wherein R⁶ and R⁷ represent -Cl and these compounds wherein R¹ is a chloro substituent and R³ is a -CF₃ group.

Still more preferred are compounds wherein R¹, R⁶ and R⁷ represent -Cl or wherein R⁶ and R⁷ represent -Cl and R³ is a -CF₃ group.

Most preferred are the 2-phenylquinoxaline derivatives wherein R¹, R⁶ and R⁷ represent -Cl and R³ represents -CF₃.

Furthermore, the compounds belonging to one of the substructures (II) - (VII) of general formula (I) are preferred:

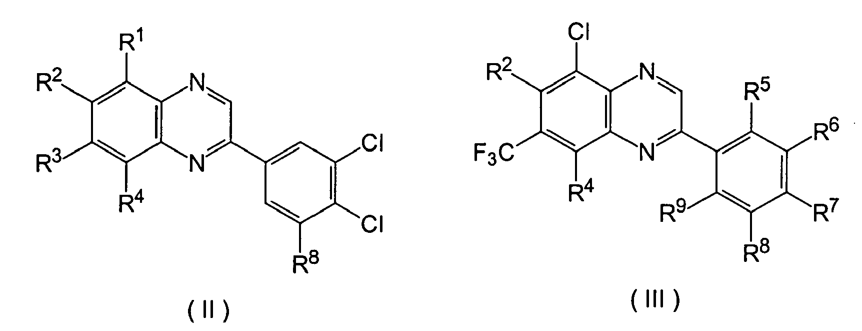

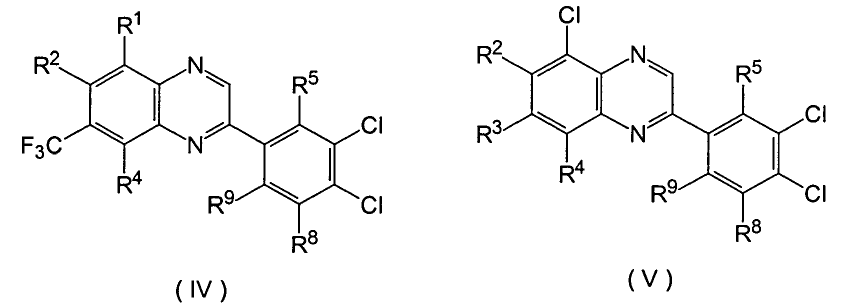

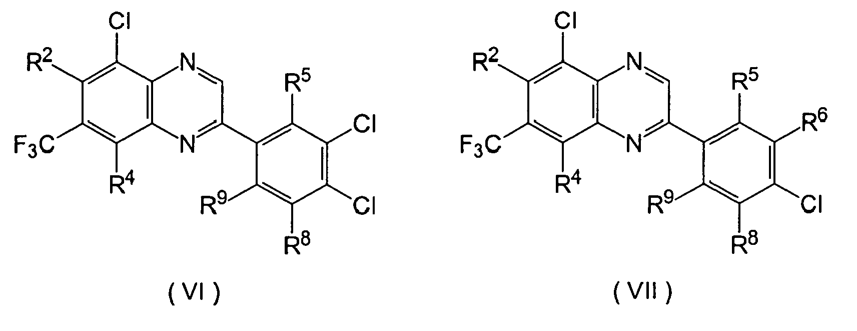

The 2-phenylquinoxaline derivatives disclosed herein are especially useful for the treatment of cancers and proliferative diseases including tumor growth and metastases. As proliferative diseases or cancer types the following can be mentioned:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

In further preferred embodiments of the present invention, at least one compound of general formula (I) - (VII) is applied in combination with chemotherapy or any other radiotherapy for the treatment of cancer.

Furthermore, it is preferred to use at least one compound of general formula (I) - (VII) in combination with a cytostatic and/or cytotoxic drug such as actinomycin D, aminoglutethimide, amsacrin, anastrozol, antagonists of purin and pyrimidin bases, anthracycline, aromatase inhibitors, asparaginase, antiestrogenes, bexaroten, bleomycin, buselerin, busulfan, camptothecin derivates, capecitabin, carboplatin, carmustin, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustin, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurin, methotrexat, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, tioguanin, topoisomerase inhibitors, topotecan, treosulfan, tretinoin, triptorelin, trofosfamide, vinblastin, vincristin, vindesin, vinorelbin, antibiotics with cytotoxic activities.

Still another aspect of the present invention relates to pharmaceutical compositions comprising at least one compound according to general formula (I) - (VII) as an active ingredient, together with at least one pharmaceutically acceptable carrier, excipient, binders, disintegrates, glidents, diluents, lubricants, coloring agents, sweetening agents, flavoring agents, preservatives or the like. The pharmaceutical compositions of the present invention can be prepared in a conventional solid or liquid carrier or diluents and a conventional pharmaceutically-made adjuvant at suitable dosage level in a known way. The preferred preparations are adapted for oral application. These administration forms include, for example, pills, tablets, film tablets, coated tablets, capsules, liposomal formulations, micro- and nano-formulations, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient. Preparations for oral use are preferred.

The pharmaceutical compositions according to the present invention containing at least one compound according to the present invention and/or a pharmaceutically acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. The pharmaceutical formulation may further contain a cytotoxic and/or cytostatic drug. Other suitable formulations are gels, elixirs, dispersable granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatine, corn sweeteners, natural and synthetic gums such as acacia, sodium alginate, carboxymethylcellulose, polyethylene glycol and waxes, sugars such as sucrose, starches derived from wheat corn rice and potato, natural gums such as acacia, tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate.

Lubricants such as boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine may be used within the pharmaceutical preparations.

Disintegrating agents (disintegrates) such as starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures can be used.

Furthermore, coloring agents, sweetening agents, flavoring agents and/or preservatives may be present in the pharmaceutical formulations.

Glidents which can be used are for example silicon dioxide and talc; suitable adsorbent are clay, aluminum oxide.

Suitable diluents are water or water/propylene glycol solutions for parenteral injections, juice, sugars such as lactose, sucrose, mannitol, and sorbitol, starches derived from wheat, corn rice, and potato, and celluloses such as microcrystalline cellulose.

### Description of figures:

- Figure 1: shows the potency study of 5-chloro-2-(3,4-dichlorophenyl)-7-(trifluoromethyl)quinoxaline. The inhibitory effect of 5-chloro-2-(3,4-dichlorophenyl)-7-(trifluoromethyl)quinoxaline on the in vitro ATPase activity of MPP1 was determined by using a malachite green assay,
- Figure 2: shows *in vitro* specifity studies of 5-chloro-2-(3,4-dichlorophenyl)-7-(trifluoromethyl)quinoxaline. The inhibitory effect of 5-chloro-2-(3,4-dichlorophenyl)-7-(trifluoromethyl)quinoxaline on the ATPase activity of the indicated kinesins was deteremined using a malachite-green assay,

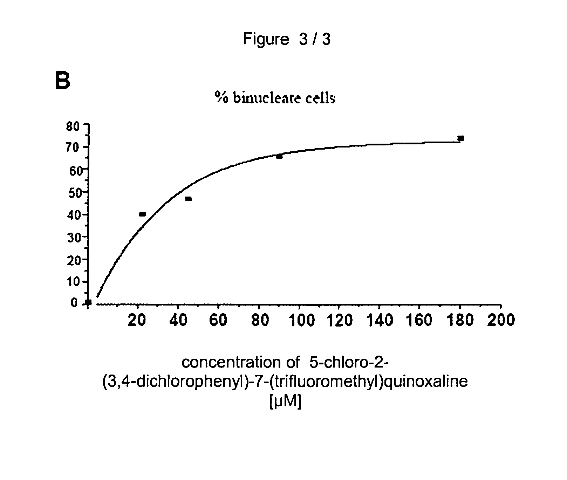

- Figure 3: shows a test concerning a cellular phenotype of 5-chloro-2-(3,4-dichlorophenyl)-7-(trifluoromethyl)quinoxaline.

### EXPERIMENTAL PART:

### Malachite-green based in vitro ATPase assay:

The 2-phenylquinoxaline derivatives were measured be means of a malachite-green based (Itaya K. and UI, M. (1966), A new micromethod for the colorimetric determination of inorganic phosphate; Clin. Chim. Acta, 14: 362 - 366) in vitro ATPase assay. For this assay the microtubule-stimulated ATPase activity of a His-tagged human MPP1 construct (Aa 1-533) in the presence of small molecules or DMSO as a solvent control were measured. These studies are done in 384-well plates in which individual compounds of a large compound collection are transferred into each well using a liquid handling robot.

### Potency study:

The potency of 5-chloro-2-(3,4-dichlorophenyl)-7-(trifluoromethyl)quinoxaline as a representative example of the 2-phenylquinoxaline derivatives of general formula (I) was determined by measuring MPP1's microtubule-stimulated ATPase activity in vitro in the presence of 5-chloro-2-(3,4-dichlorophenyl)-7-(trifluoromethyl)-quinoxaline or DMSO as a solvent control. For these studies a malachite green assay which allows the quantification of free phosphate (Pi) in solution was used.

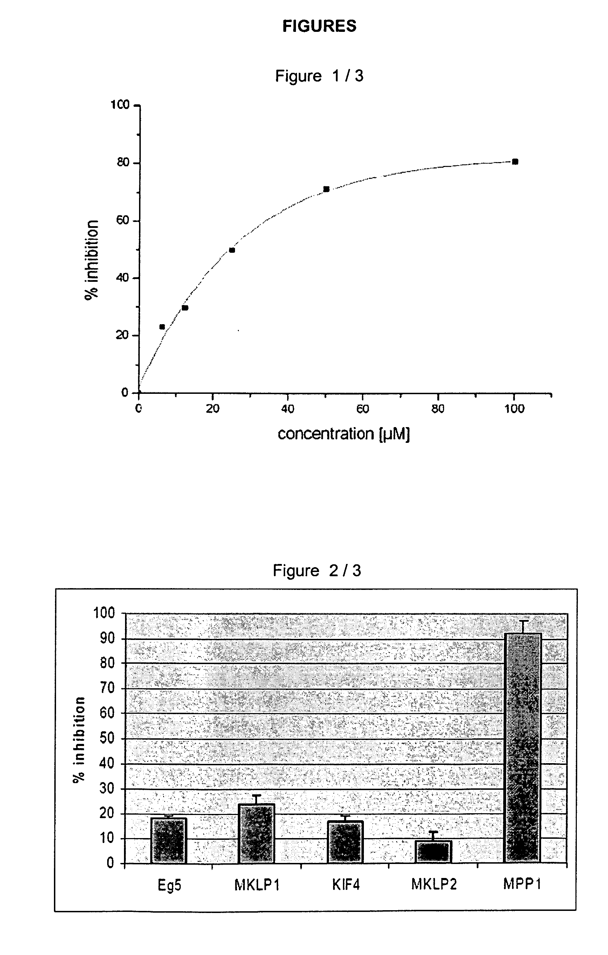

It was found that 5-chloro-2-(3,4-dichlorophenyl)-7-(trifluoromethyl)quinoxaline inhibits the in vitro ATPase activity of MPP1 with an IC50 of approximately 40 µM (cf. Fig. 1).

### Specificity:

The specificity of 5-chloro-2-(3,4-dichlorophenyl)-7-(trifluoromethyl)quinoxaline was determined by measuring its effect on the microtubule-stimulated in vitro ATPase activities of the kinesins Eg5, Kif4, Mklp1, and Mklp2.

The compound 5-chloro-2-(3,4-dichlorophenyl)-7-(trifluoromethyl)quinoxaline has no significant effect on the in vitro ATPase activities of Eg5, Kif4, Mklp1, and Mklp2 (cf. Fig. 2).

### Cellular phenotype:

Concerning the study the function of MPP1 in living cells, it was tested whether 5-chloro-2-(3,4-dichlorophenyl)-7-(trifluoromethyl)quinoxaline induces a phenotype similar to the one obtained by RNAi-mediated depletion of MPP1. For these studies we treated synchronized BSC-1 (African green monkey) cells with 5-chloro-2-(3,4-dichlorophenyl)-7-(trifluoromethyl)quinoxaline or DMSO as a solvent control.

BSC-1 cells were released from thymidine-block for three hours and then treated with 50µM or the indicated concentrations of 5-chloro-2-(3,4-dichlorophenyl)-7-(trifluoromethyl)quinoxaline for 18 hours. Treated cells were fixed with formaledehyde-containing buffer and the DNA and microtubule cytoskeleton were visualized with Hoechst and FITC-labled anti-tubulin antibody, respectively. The number of binucleated cells was determined by manual counting of at least 200 cells per concentration.

It was found that the compound 5-chloro-2-(3,4-dichlorophenyl)-7-(trifluoromethyl)quinoxaline induces a binucleate phenotype in BSC-1 with an EC50 of about 50 µM. Similarly, 5-chloro-2-(3,4-dichlorophenyl)-7-(trifluoromethyl)quinoxaline induces a binucleate phenotype in Hela S3 cells (cf.
Fig. 3).

### Specificity in vivo:

For the specificity *in vivo* studies, the effect of 5-chloro-2-(3,4-dichlorophenyl)-7-(trifluoromethyl)quinoxaline on cellular processes known to be independent of MPP1 function, e.g. golgi/lysosome localization, actin/microtubule cytoskeleton in interphase, protein synthesis, S to M-phase cell cycle progression, and wound healing was investigated.

The compound 5-chloro-2-(3,4-dichlorophenyl)-7-(trifluoromethyl)quinoxaline had no observable effect on the tested cellular processes.

## Claims

1. Compounds having the general formula (I): 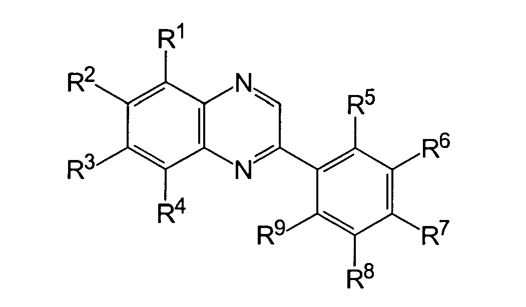 wherein
R¹, R², R³,R⁴,R⁵,R⁸, and R⁹ are independently of each other selected from R¹⁰ - R¹⁸, -CH₂-R¹⁹, -C₂H₄-R²⁰, -C₃H₆-R²¹, -C₄H₈-R²², -C₅H₁₀-R²³, -C₆H₁₂-R²⁴, -C₇H₁₄-R²⁵, -CHR²⁶R²⁷, -CR²⁸R²⁹R³⁰, -CHR³¹-CHR³²R³³, -CR³⁴R³⁵-CR³⁶R³⁷R³⁸, -CHR³⁹-CHR⁴⁰-CHR⁴¹R⁴² -CR⁴³R⁴⁴-CR⁴⁵R⁴⁶-CR⁴⁷R⁴⁸R⁴⁹.
R¹⁰ - R⁴⁹ represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC (CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cydo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NH-cyclo-C₃H₅. -NH-CS-NHC₃H₇, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-C(=NH)-NH₂, -NH-CS-NHC₂H₅, -NH-CS-NHCH₃, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F -CHF₂, -CF₃, -CH₂Cl, -CHCl₂, -CCl₃, -CH₂Br -CHBr₂, -CBr₃, -CPh₃, -CH₂-CH₂F -CH₂-CHF₂, -CH₂-CF₃, -CH₂-CH₂Cl, -CH₂-CHCl₂, -CH₂-CCl₃, -CH₂-CH₂Br -CH₂-CHBr₂, -CH₂-CBr₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH₂-C(CH₃)₂-C₂H₅, -CH(CH₃)-CH(CH₃)-C₂H₅, -CH₂-CH(CH₃)-CH(CH₃)₂, -C(CH₃)₂-C₃H₇, -C(CH₃)₂-CH(CH₃)₂, -C₂H₄-C(CH₃)₃, -CH(CH₃)-C(CH₃)₃, -C₇H₁₅, -C₈H₁₇, -C₉H₁₉, -C₁₀H₂₁, -Ph, -CH₂-Ph, -C₂H₄-Ph, -C₂H₂-Ph, -C≡C-Ph, -CPh₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -C≡CH, -CH=CH-C₃H₇, -C≡C-CH₃, -CH₂-C≡CH, -C₂H₄-C≡CH, -CH₂-C≡C-CH₃, -C≡C-C₂H₅, -C₃H₆-C≡CH, -cyclo-C₃H₅, -cyclo-C₄H₇, -cyclo-C₅H₉, -cyclo-C₆H₁₁;
R⁶ and R⁷ represent -Cl; and
at least one of the substituents R⁵, R⁸, and R⁹ is different from hydrogen;
and stereoisomeric forms, prodrugs, solvates, hydrates and/or pharmaceutically acceptable salts thereof.

2. Compound according to claim 1 wherein R¹ represents -Cl.

3. Compound according to claim 1 or 2 wherein R³ represents -CF₃.

4. Compound according to any previous claim for use as a pharmaceutically active agent.

5. Use of a compound having the general formula (I): 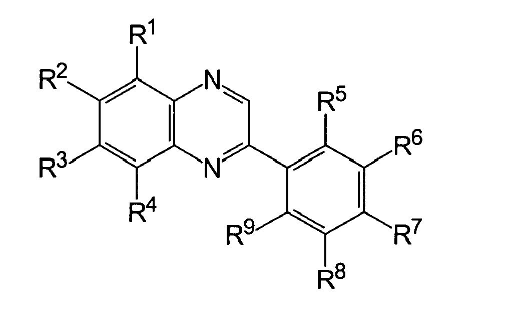 wherein
R¹, R², R³, R⁴, R⁵, R⁶, R⁷, R⁸, and R⁹ are independently of each other selected from R¹⁰ - R¹⁸, -CH₂-R¹⁹, -C₂H₄-R²⁰, -C₃H₆-R²¹, -C₄H₈-R²², -C₅H₁₀-R²³, -C₆H₁₂-R²⁴, -C₇H₁₄-R²⁵, -CHR²⁶R²⁷, -CR²⁸R²⁹R³⁰, -CHR³¹-CHR³²R³³, -CR³⁴R³⁵-CR³⁶R³⁷R³⁸, -CHR³⁹-CHR⁴⁰-CHR⁴¹R⁴², -CR⁴³R⁴⁴-CR⁴⁵R⁴⁶-CR⁴⁷R⁴⁸R⁴⁹
R¹⁰ - R⁴⁹ represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NH-cyclo-C₃H₅, -NH-CS-NHC₃H₇, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-C(=NH)-NH₂, -NH-CS-NHC₂H₅, -NH-CS-NHCH₃, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F -CHF₂, -CF₃, -CH₂Cl, -CHCl₂, -CCl₃, -CH₂Br -CHBr₂, -CBr₃, -CPh₃, -CH₂-CH₂F -CH₂-CHF₂, -CH₂-CF₃, -CH₂-CH₂Cl, -CH₂-CHCl₂, -CH₂-CCl₃, -CH₂-CH₂Br -CH₂-CHBr₂, -CH₂-CBr₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH₂-C(CH₃)₂-C₂H₅, -CH(CH₃)-CH(CH₃)-C₂H₅, -CH₂-CH(CH₃)-CH(CH₃)₂, -C(CH₃)₂-C₃H₇, -C(CH₃)₂-CH(CH₃)₂, -C₂H₄-C(CH₃)₃, -CH(CH₃)-C(CH₃)₃, -C₇H₁₅, -C₈H₁₇, -C₉H₁₉, -C₁₀H₂₁, -Ph, -CH₂-Ph, -C₂H₄-Ph, -C₂H₂-Ph, -C≡C-Ph, -CPh₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -C≡CH, -CH=CH-C₃H₇, -C≡C-CH₃, -CH₂-C≡CH, -C₂H₄-C≡CH, -CH₂-C≡C-CH₃, -C≡C-C₂H₅, -C₃H₆-C≡CH. -cyclo-C₃H₅, -cyclo-C₄H₇, -cyclo-C₅H₉, -cyclo-C₆H₁₁;
and stereoisomeric forms, prodrugs, solvates, hydrates and/or pharmaceutically acceptable salts thereof for prophylaxis and/or treatment of cancers and proliferative diseases.

6. Use of a compound according to claim 5 as an inhibitor of the mitotic kinesin MPP1.

7. Use of a compound according to claim 5 or 6 wherein R⁶ and/or R⁷ represent(s) -Cl.

8. Use of a compound according to any one of claims 5-7 wherein R¹ represents -Cl.

9. Use of a compound according to any one of claims 5 - 8 wherein R³ represents -CF₃.

10. Use of a compound according to any one of claims 5 - 9, wherein the proliferative disease or cancer is selected from the group comprising:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

11. Use of a compound according to any one of claims 5 - 10 in combination with chemotherapy or radiotherapy.

12. Use of a compound according to any one of claims 5 - 11 in combination with actinomycin D, aminoglutethimide, amsacrin, anastrozol, antagonists of purin and pyrimidin bases, anthracycline, aromatase inhibitors, asparaginase, antiestrogenes, bexaroten, bleomycin, buselerin, busulfan, camptothecin derivates, capecitabin, carboplatin, carmustin, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustin, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurin, methotrexat, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, tioguanin, topoisomerase inhibitors, topotecan, treosulfan, tretinoin, triptorelin, trofosfamide, vinblastin, vincristin, vindesin, vinorelbin, antibiotics with cytotoxic activities.

13. Pharmaceutical composition comprising at least one compound of general formula (I) as an active ingredient, together with at least one pharmaceutically acceptable carrier, excipient and/or diluents.
